# EUROPEAN PATENT APPLICATION

(11) **EP 4 446 404 A1**
(43) Date of publication of application: **16.10.2024**
(21) Application number: 21967317.5
(22) Date of filing: 08.12.2021
(51) Int. Cl.: C12N 1/20, A61K 35/742, A61P 25/00, A61P 1/14, A23L 33/135, A23K 10/16, C12R 1/225

(54) **COMPOSITION FOR PREVENTION OR TREATMENT OF COGNITIVE DYSFUNCTION AND INTESTINAL DYSFUNCTION, COMPRISING LACTOBACILLUS PARACASEI NK112, CUSCUTA AUSTRALIS EXTRACT, AND CUSCUTA JAPONICA EXTRACT**

(30) Priority: 06.12.2021 KR 20210173108
(71) Applicant: Mthera Pharma Co., Ltd., Seoul 07793 (KR)
(72) Inventor: SOHN, Mi Won, Yongin-si Gyeonggi-do 16882 (KR); CHOI, Jin Gyu, Seoul 03505 (KR); KIM, Sinyeon, Seoul 07063 (KR); PARK, Sang Cheol, Seoul 03115 (KR); KIM, Dong Hyun, Seoul 02823 (KR); KIM, Sun Yeou, Seoul 06280 (KR); HONG, Sung Min, Chungju-si Chungcheongbuk-do 27356 (KR); OH, Myung Sook, Seoul 06291 (KR); JU, In Gyoung, Seoul 02451 (KR); LEE, Choong Hwan, Seoul 05813 (KR)
(74) Representative: Forrest, Stuart
(86) International application number: PCT/KR2021/018529
(87) International publication number: WO 2023/106444

(57) **Abstract**

The present invention relates to a novel *Lactobacillus paracasei* NK112 strain that has the effect of preventing or treating cognitive dysfunction and intestinal dysfunction. In addition, the present invention relates to a composition for prevention, treatment, or alleviation of cognitive dysfunction and intestinal dysfunction, comprising a *Lactobacillus paracasei* NK112 strain, a *Cuscuta australis* R.Br. extract, and a *Cuscuta japonica* Choisy extract. The strain and the composition, of the present invention, exhibit efficacy in increasing the expression of brain-derived neurotrophic factor (BDNF) and nerve growth factor (NGF), alleviating intestinal inflammation, and the like, and thus can be effectively used in the prevention or treatment of cognitive dysfunction and intestinal dysfunction.

## Description

### Technical Field

The present invention relates to a novel *Lactobacillus paracasei* NK112 strain.

In addition, the present invention relates to a composition for prevention, treatment, or alleviation of cognitive dysfunction and intestinal dysfunction, comprising a *Lactobacillus paracasei* NK112 strain, a *Cuscuta australis* R.Br. extract, and a *Cuscuta japonica* Choisy extract.

### Background Art

As humanity gradually develops into an affluent society, lifestyle habits are rapidly becoming westernized. In addition, due to a westernized diet centered on meat and fat, irregular meals, excessive drinking, lack of exercise, excessive stress, and exposure to harmful environments, diseases such as intestinal diseases such as colitis and disturbances in the intestinal bacterial community are increasing. These intestinal diseases and an increase in intestinal bacterial community imbalance rapidly increase cognitive dysfunction such as dementia, Parkinson's disease, depression, and anxiety.

Degenerative brain diseases such as dementia refer to diseases in which the ability to remember or think gradually declines due to neurodegeneration, reaching a level that affects daily life. As we enter an aging society, it is emerging as one of the main causes of medical, economic, and social burden. As of 2015, there are approximately 47 million patients worldwide, and the prevalence of these diseases in the population aged 60 or older is estimated to be approximately 7% (The global prevalence of dementia: a systematic review and metaanalyisis, Alzheimers Dement, 2013, 63-75).

In addition to dementia, cognitive dysfunction may occur due to diseases and symptoms that are common in modern life, such as high blood pressure and lack of exercise. In particular, depression, which has recently been rapidly increasing in the number of patients of all ages, is known to have the highest correlation with cognitive dysfunction. Therefore, it is necessary to develop methods to alleviate cognitive dysfunction and protect nerve cells as a means to lead a healthy daily life.

In the case of diseases such as cognitive dysfunction that require continuous management while taking drugs for a long period of time, reducing side effects is one of important tasks. For example, donepezil, which is commonly used to treat dementia in Korea, can cause side effects such as muscle pain and loss of appetite, as well as hallucinations and abdominal disorders in severe cases, so the development of a new therapeutic agent for dementia is urgently needed. Therefore, the development of natural medicines with fewer side effects than synthetic medicines in diseases related to cognitive dysfunction can be of great help to patients.

*Cuscuta australis* R.Br. and *Cuscuta japonica* Choisy are annual climbing plants belonging to the family Convolvulaceae and are also called Cuscutae Semen or dodder seed. Dried ripe seeds of *Cuscuta australis* R.Br. are used to replenish positive energy and negative energy, prevent semen from leaking out, control urination, and brighten the eyes. It has been reported that the ingredients contained in *Cuscuta australis* R.Br. include quercetin, kaempferol, isorhamnetin, caffeic acid, thymidine, sesamin, and taraxanthin (A Review on Phytoconstituents and Biological activities of Cuscuta species. Biomed Pharmacother. 2017 Aug; 92:772-795). The ingredients contained in *Cuscuta japonica* Choisy include resinous glycosides, sugars, sterols, triterpenoids, gibberellin, alkaloids, cardiac glycosides, steroid saponins, and the like.

Meanwhile, many bacteria live in the body, and the number of bacteria is greater than the number of normal cells. These bacteria are divided into beneficial bacteria that help the health of our intestines and harmful bacteria that are harmful to our health. Our body can maintain health when beneficial bacteria such as Lactobacillus and Bifidobacterium inhabit the digestive tract as the dominant bacteria (Gut/brain axis and the microbiota. The Journal of clinical investigation, 2015, 125(3), 926-938). On the other hand, it is known that when brain disease occurs, harmful microorganisms such as Escherichia coli and Proteus mirabilis increase in the intestinal bacterial flora, which causes overexpression of α-synuclein in intestinal cells and activation of NF-κB (nuclear factor kappa-light-chain-enhancer of activated B cells), thereby accelerating the progression of Parkinson's disease and Alzheimer's disease (Oral administration of Proteus mirabilis damages dopaminergic neurons and motor functions in mice. Scientific reports, 2018, 8(1), 1275).

Live microorganisms that improve the intestinal microbial environment and have a beneficial effect on the host's health are collectively called probiotics. When ingested into the body, lactic acid bacteria coexist in the digestive system and break down fiber and complex proteins, contributing to important nutrients, so they are used as probiotics. It has been reported that lactic acid bacteria can maintain and improve intestinal bacterial flora, have anti-diabetic effects, suppress colitis, and improve the immune system (Prophylactic and therapeutic uses of probiotics: a review. Journal of the American Dietetic Association, 2001, 101(2), 229-241). Research is being actively conducted to develop lactic acid bacteria with various physiologically active effects and apply them as materials for medicines or health functional foods (Korean Patent Registration No. 10-1476236, etc.), but there have been no cases of it being developed as a therapeutic agent for chronic diseases such as cognitive dysfunction.

In this regard, the inventors of the present invention conducted continuous research to develop methods for the prevention and treatment of cognitive dysfunction and intestinal dysfunction. As a result, the present inventors found excellent cognitive and intestinal function improvement effects when using a novel lactic acid bacteria *Lactobacillus paracasei* NK112, a *Cuscuta australis* R.Br. extract, and a *Cuscuta japonica* Choisy extract, thereby completing the present invention.

### Detailed Description of Invention

### Technical Problem

An object of the present invention is to provide a novel *Lactobacillus paracasei* NK112 strain that has the effect of preventing or treating cognitive dysfunction and intestinal dysfunction.

In addition, another object of the present invention is to provide a composition for prevention, treatment, or alleviation of cognitive dysfunction and intestinal dysfunction, comprising a *Lactobacillus paracasei* NK112 strain, a *Cuscuta australis* R.Br. extract, and a *Cuscuta japonica* Choisy extract.

### Solution to Problem

In order to achieve the above object, the present invention provides a novel *Lactobacillus paracasei* NK112 strain deposited under the accession number: KCCM 125 66P.

In one embodiment, the *Lactobacillus paracasei* NK112 strain has the effect of preventing or treating cognitive dysfunction or intestinal dysfunction.

In addition, the present invention provides a composition for prevention, treatment, or alleviation of cognitive dysfunction or intestinal dysfunction, comprising at least one selected from the group consisting of a *Lactobacillus paracasei* NK112 strain, a *Cuscuta australis* R.Br. extract, and a *Cuscuta japonica* Choisy extract.

The composition may be a pharmaceutical composition, a food composition, or an animal feed composition.

In one embodiment, the composition may comprise a *Lactobacillus paracasei* NK112 strain, a *Cuscuta australis* R.Br. extract, and a *Cuscuta japonica* Choisy extract.

In one embodiment, the composition may comprise a *Cuscuta australis* R.Br. extract, a *Cuscuta japonica* Choisy extract, and a *Lactobacillus paracasei* NK112 strain at a weight ratio of 0.3 to 30 : 1 : 0.05 to 5, at a weight ratio of 1 to 6 : 1 : 0.1 to 1, or at a weight ratio of 2 to 4 : 1 : 0.3 to 7, and preferably, may comprise at a weight ratio of 3 : 1 : 0.5.

In one embodiment, the composition may promote the expression of brain-derived neurotrophic factor (BDNF).

In one embodiment, the composition may promote the expression of nerve growth factor (NGF).

In one embodiment, the composition may be an extract of a mixture of *Cuscuta australis* R.Br. and *Cuscutajaponica* Choisy.

In one embodiment, the extract may be extracted with a solvent selected from the group consisting of water (distilled water), C1 to C6 alcohol, ethyl acetate, acetone, and mixed solvents thereof, and may be preferably extracted with distilled water.

In one embodiment, the extract may be extracted with a solvent in an amount 10 times to 50 times the weight of *Cuscuta australis* R.Br. and *Cuscuta japonica* Choisy, and preferably, may be extracted with a solvent in an amount 20 times to 30 times the weight of *Cuscuta australis* R.Br. and *Cuscutajaponica* Choisy.

In one embodiment, the extract may be reflux-extracted or ultrasonic-extracted from *Cuscuta australis* R.Br. and *Cuscuta japonica* Choisy, and preferably, may be reflux-extracted.

In one embodiment, the extract may be an extract of the raw material (whole crude drug) or powder (powdered crude drug) of *Cuscuta australis* R.Br. and *Cuscuta japonica* Choisy, and preferably, may be an extract of the powder of *Cuscuta australis* R.Br. and *Cuscutajaponica* Choisy.

In one embodiment, the *Lactobacillus paracasei* NK112 strain may be a heat-killed cell, a cell wall fraction, or a cytoplasmic fraction of *Lactobacillus paracasei* NK112.

In one embodiment, the cognitive dysfunction of the present invention may be at least one selected from the group consisting of Alzheimer's disease, schizophrenia, Parkinson's disease, Huntington's disease, Pick's disease, Creutzfeldt-Jakob disease, depression, anxiety, stroke, cerebral ischemia, encephalitis, amnesia, traumatic brain injury, Wernicke Korsakoff syndrome, epilepsy, seizure, hippocampal sclerosis, cerebral senescence, dementia, frontotemporal degeneration, normal pressure hydrocephalus, memory loss, and learning disability.

In one embodiment, the intestinal dysfunction of the present invention may be at least one selected from the group consisting of inflammatory bowel disease, intestinal peristalsis disorder, intestinal obstruction, colon atony, irritable bowel syndrome, dyspepsia, abdominal distension, diarrhea, and constipation.

### Effects of Invention

A *Lactobacillus paracasei* NK112 strain according to the present invention, and a composition comprising the strain, a *Cuscuta australis* R.Br. extract, and a *Cuscuta japonica* Choisy extract exhibit efficacy in increasing the expression of brain-derived neurotrophic factor (BDNF) and nerve growth factor (NGF), alleviating intestinal inflammation, and the like, and thus can be effectively used in the prevention or treatment of cognitive dysfunction and intestinal dysfunction.

### Brief Description of Drawings

Figures 1a and 1b show the spontaneous alternation of mice in a Y-maze task (1a) and the latency time in a bright room due to fear memory in a passive avoidance test (1b) when the NK112 strain was administered to mice.
Figures 2a and 2b show the amount of nerve growth factor (NGF) produced when treated with a mixed composition of a *Cuscuta australis* R.Br. extract, a *Cuscuta japonica* Choisy extract, and a NK112 strain.
Figure 3 shows the viability of nerve cells when treated with a mixed composition of a *Cuscuta australis* R.Br. extract, a *Cuscuta japonica* Choisy extract, and a NK112 strain.
Figures 4a to 4c show the spontaneous alternation of mice in a Y-maze task (4a), the recognition index in a novel object recognition test (4b), and the latency time in a bright room due to fear memory in a passive avoidance test (4c) when the mice were treated with a mixed composition of a *Cuscuta australis* R.Br. extract, a *Cuscuta japonica* Choisy extract, and a NK112 strain.
Figures 5a and 5b show the observation photograph of the brain rotation area of the hippocampus (5a) and the amount of synaptic protein (5b) when the mice were treated with a mixed composition of a *Cuscuta australis* R.Br. extract, a *Cuscuta japonica* Choisy extract, and a NK112 strain.
Figures 6a to 6c show the observation photograph of hippocampal dentate gyrus cells (6a), the number of neuroblasts (6b), and the length of neurites (6c) when the mice were treated with a mixed composition of a *Cuscuta australis* R.Br. extract, a *Cuscuta japonica* Choisy extract, and a NK112 strain.
Figures 7a to 7d show changes in intestinal length and inflammatory index values when the mice were treated with a mixed composition of a *Cuscuta australis* R.Br. extract, a *Cuscuta japonica* Choisy extract, and a NK112 strain.

### Best Mode for Carrying out the Invention

Hereinafter, with reference to the accompanying drawings, embodiments and examples of the present invention will be described in detail so that those of ordinary skill in the art to which the present invention belongs can easily carry out. However, the present invention may be implemented in various forms and is not limited to the embodiments and examples described herein.

Throughout the present specification, when a certain part "includes" a certain component, it means that other components may be further included, rather than excluding other components, unless otherwise stated.

The present invention relates to a novel lactic acid bacterium, a *Lactobacillus paracasei* NK112 strain.

In addition, the present invention relates to a composition for prevention, treatment, or alleviation of cognitive dysfunction or intestinal dysfunction, comprising at least one selected from the group consisting of a *Lactobacillus paracasei* NK112 strain, a *Cuscuta australis* R.Br. extract, and a *Cuscuta japonica* Choisy extract.

The composition has the efficacy in promoting the expression of brain-derived neurotrophic factor (BDNF) and nerve growth factor (NGF). Nerve growth factor (NGF) binds to the TrkA (tropomyosin receptor kinase A) receptor and activates TrkA into pTrkA, and the complex of nerve growth factor (NGF) and pTrkA moves to the cell body and affects nerve regeneration. In addition, it activates cAMP-response element binding protein (CREB), which plays an important role in regulating cell cycle, neurite outgrowth, and synaptic plasticity. Brain-derived neurotrophic factor (BDNF) is one of the nerve growth promoting factors and is known to play an important role in the regulation of neurotransmitters and neuroplasticity. Therefore, increased expression of nerve growth factor or brain-derived neurotrophic factor means that it has a nerve regeneration effect.

The extract of the present invention can be prepared by a production method comprising the following steps, but is not limited thereto:
1) adding an extraction solvent to *Cuscuta australis* R.Br. and *Cuscuta japonica* Choisy to extract them;
2) filtering the extract of step 1); and
3) concentrating the filtered extract of step 2) under reduced pressure and then drying it.

In the above method, conventional methods in the art such as reflux extraction, ultrasonic extraction, stirring extraction, filtration, hot water extraction, and immersion extraction can be used as the extraction method.

In the above method, the concentration under reduced pressure in step 3) may be performed using a vacuum reduced pressure concentrator or a vacuum rotary evaporator, but is not limited thereto. In addition, drying may include reduced pressure drying, vacuum drying, boiling drying, spray drying, or freeze drying, but is not limited thereto.

The food composition of the present invention may be a health functional food. The "health functional food" refers to a food manufactured and processed using raw materials or ingredients having useful functionality for the human body, and "functionality" refers to regulating nutrients for the structure and function of the human body or ingesting nutrients for the purpose of obtaining useful effects for health purposes such as physiological functions and the like.

Hereinafter, the present invention will be described in more detail through the examples, but the following examples are for illustrative purposes only and are not intended to limit the scope of the present invention.

### [Example 1]

### Isolation and identification of lactic acid bacteria

### 1-1. Isolation of lactic acid bacteria from human feces

Human feces were placed in GAM liquid medium (GAM broth, Nissui Pharmaceutical, Japan) and suspended. Thereafter, the supernatant was taken, transplanted onto BL agar medium (Nissui Pharmaceutical, Japan), and cultured anaerobically at 37 °C for about 48 hours, and then the strains that formed colonies were isolated.

### 1-2. Identification of isolated lactic acid bacteria

In order to confirm physiological properties and phylogenetic positions of the isolated strains, the nucleotide sequences were analyzed. As a result, they showed 90-99% nucleotide sequence similarity with strains of the genus Lactobacillus, indicating that they were a strain of the genus Lactobacillus.

Among the above strains, the nucleotide sequence of the strain having the 16S rDNA nucleotide sequence of SEQ ID NO: 1 below was analyzed using Gene Bank's BLAST program. As a result, it was confirmed that it was a novel strain that has 99% homology to the 16S rDNA sequence of the *Lactobacillus paracasei* strain. It was designated as *Lactobacillus paracasei* NK112. The above strain was deposited on July 18, 2019 at the Korean Culture Center of Microorganisms, and was given accession number: KCCM12566P.

| SEQ ID NO: 1 | NK112 strain 168 rDNA |
|---|---|
| ACCTGCCCTTAAGTGGGGGATAACATTTGGAAACAGATGCTAATACCGCATAGATCCAAG AACCGCATGGTTCTTGGCTGAAAGATGGCGTAAGCTATCGCTTTTGGATGGACCCGCGGCGTATT AGCTAGTTGGTGAGGTAATGGCTCACCAAGGCGATGATACGTAGCCGAACTGAGAGGTTGATCGG CCACATTGGGACTGAGACACGGCCCAAACTCCTACGGGAGGCAGCAGTAGGGAATCTT CCACAATGGACGCAAGTCTGATGGAGCAACGCCGCGTGAGTGAAGAAGGCTTTCGGGTCGTAAA ACTCTGTTGTTGGAGAAGAATGGTCGGCAGAGTAACTGTTGTCGGCGTGACGGTATCCA ACCAGAAAGCCACGGCTAACTACGTGCCAGCAGCCGCGGTAATACGTAGGTGGCAAGCGTTATC CGGATTTATTGGGCGTAAAGCGAGCGCAGGCGGTTTTTTAAGTCTGATGTGAAAGCCCTC GGCTTAACCGAGGAAGCGCATCGGAAACTGGGAAACTTGAGTGCAGAAGAGGACAGTGGAACT CCATGTGTAGCGGTGAAATGCGTAGATATATGGAAGAACACCAGTGGCGAAGGCGGCTG TCTGGTCTGTAACTGACGCTGAGGCTCGAAAGCATGGGTAGCGAACAGGATTAGATACCCTGGTA GTCCATGCCGTAAACGATGAATGCTAGGTGTTGGAGGGTTTCCGCCCTTCAGTGCCGCA GCTAACGCATTAAGCATTCCGCCTGGGGAGTACGACCGCAAGGTTGAAACTCAAAGGAATTGAC GGGGGCCCGCACAAGCGGTGGAGCATGTGGTTTAATTCGAAGCAACGCGAAGAACCTTA CCAGGTCTTGACATCTTTTGATCACCTGAGAGATCAGGTTTCCCCTTCGGGGGCAAAATGACAGG TGGTGCATGGTTGTCGTCAGCTCGTGTCGTGAGATGTTGGGTTAAGTCCCGCAACGAGCG CAACCCTTATGACTAGTTGCCAGCATTTAGTTGGGCACTCTAGTAAGACTGCCGGTGACAAACCG GAGGAAGGTGGGGATGACGTCAAATCATCATGCCCCTTATGACCTGGGCTACACACGTGC TACAATGGATGGTACAACGAGTTGCGAGACCGCGAGGTCAAGCTAATCTCTTAAAGCCATTCTCA GTTCGGACTGTAGGCTGCAACTCGCCTACACGAAGTCGGAATCGCTAGTAATCGCGGATC AGCACGCCGCGGTGAATACGTTCCCGGGCCTTGTACACACCGCCCGTCACACCATGAGAGTTTGT AACACCCGAAGCCGGTGGCGTAACCCTTTTAGGGAGCGAGCCGTCTAAGGTGGGACAAT GATTAGGGTGAAGTCTA | |

### 1-3. Biochemical identification using API kits

The physiological properties of the *Lactobacillus paracasei* NK112 strain were analyzed by a sugar fermentation test using the API 20 strep Kit (BioMerieux's, USA), and the results are shown in Table 1 below. In Table 1 below, "+" indicates a case where carbon source availability is positive, and "-" indicates a case where carbon source availability is negative. As a result of the analysis, it was found that NK112 showed the most similar biochemical properties to *Lactobacillusparacasei* at 56.5%.

**[Table 1]**

| Carbon source | *Lactobacillus paracasei* NK112 |
|---|---|
| glycerol | - |
| erythritol | - |
| D-arabinose | - |
| L-arabinose | - |
| D-ribose | + |
| D-xylose | - |
| L-xylose | - |
| D-adonitol | - |
| methyl-β-D-xylopyranoside | - |
| D-galactose | + |
| D-glucose | + |
| D-fructose | + |
| D-mannose | + |
| L-sorbose | - |
| L-rhamnose | - |
| dulcitol | - |
| inositol | - |
| mannitol | + |
| sorbitol | - |
| α-methyl-D-mannoside | - |
| α-methly-D-glucoside | + |
| N-acetyl-glucosamine | + |
| amygdalin | + |
| arbutin | + |
| esculin | + |
| salicin | + |
| cellobiose | + |
| maltose | + |
| lactose | - |
| melibiose | - |
| sucrose | + |
| trehalose | + |
| inulin | - |
| melezitose | + |
| raffinose | - |
| starch | - |
| glycogen | - |
| xylitol | - |
| gentiobiose | + |
| D-turanose | + |

### [Example 2]

### Confirmation of the effect of brain-derived neurotrophic factor (BDNF) expression in nerve cells when treated with NK112 strain

Human-derived SH-SY5Y nerve cells were cultured in DMEM medium supplemented with 10% FBS and 1% antibiotics, and dispensed in a 12-well plate at 2 X 10⁶ cells/well. Thereafter, the NK112 strain (1 X 10⁵ CFU/ml) and lipopolysaccharide were added to each well at a concentration of 2 µg/ml and cultured, and the expression level of brain-derived neurotrophic factor was measured by immunoblotting. As a result, it was found that the *Lactobacillus paracasei* NK112 strain was effective in increasing the expression of brain-derived neurotrophic factor, which is decreased in aging and dementia, to a level of 10 to 30%.

### [Example 3]

### Confirmation of anti-anxiety and anti-depression efficacy of NK112 strain

In order to confirm the cognitive function improvement effect of a *Lactobacillus paracasei* NK112 strain, the following experiment was performed.

### 3-1. Production of Escherichia coli-induced cognitive dysfunction and intestinal dysfunction model

5-week-old male C57BL/6 mice having a body weight of 19 to 21 g were divided into a normal group and an experimental group, with 6 mice per group, and acclimatized to the laboratory for 1 week, and then the experiment was performed. The experimental group was administered with Escherichia coli (1 × 10⁹ CFU/mouse/day) orally once a day for 5 days, and the normal group was administered with physioligical saline.

Thereafter, 1 × 10⁹ CFU heat-treated lactic acid bacteria (heat-killed NK112), 1 × 10⁹ CFU lactic acid bacteria lysate supernatant (NK112 cytoplasmic component), 1 × 10⁹ CFU lactic acid bacteria lysate precipitate (NK112 cell wall component), or positive control group drug (fluoxetine 1 mg/kg) was administered once a day for 5 days, respectively.

In the case of heat-treated lactic acid bacteria (heat-killed NK112), the precipitate obtained by centrifuging the lactic acid bacteria culture solution in MRS medium was suspended to 1 × 10¹⁰ CFU/ml, and then heat-treated twice at 70 °C for 10 minutes each to prepare them.

For lactic acid bacteria lysate supernatant (NK112 cytoplasmic component) and precipitate (NK112 cell wall component), the lactic acid bacteria culture solution was centrifuged in MRS medium at 5,000 g for 20 minutes, and the obtained precipitate was suspended to 1 × 10¹⁰ CFU/ml and sonicated 20 times for 1 minute each. Thereafter, it was obtained by centrifugation again at 5,000 g for 20 minutes.

In order to evaluate the anti-anxiety and anti-depression efficacy of the *Lactobacillus paracasei* NK112 strain using the mouse model, an experiment was performed as follows.

### 3-2. Elevated plus maze (EPM) test

The elevated plus maze is an experimental apparatus to measure the level of anxiety, depression, or stress, etc. The elevated plus maze used in this experiment is a black flexi glass apparatus that has two open arms (30 X 7 cm) and two enclosed arms (30 X 7 cm) with 20 cm high walls, which are 50 cm above the floor and extend 7 cm from the central platform. The movements of the mouse placed in the elevated plus maze were recorded in a room with a brightness of 20 lux and a video camera installed above.

Specifically, a C57BL/6 mouse was placed facing the open arm with its head in the exact center of the elevated plus maze, and the time and number of times it spent in the open arms and the enclosed arms for 5 minutes were measured. Arm entry was recognized as entering with all four feet.

Of the total experimental time, time spent in open arms (OT) was calculated as [time spent in open arms / (time spent in open arms + time spent in enclosed arms)] X 100. Open arm entry (OE) was calculated as [open arm entry / (open arm entry + enclosed arm entry)] X 100. After completion of each behavioral experiment, the remaining odor was removed with 70% ethanol.

According to a known method of interpreting test results, a decrease in time spent in open arms (OT) and open arm entry (OE) was interpreted as indicating symptoms such as anxiety and depression.

### 3-3. Forced swimming test (FST)

A water tank with a height of 40 cm and a diameter of 20 cm was filled with water at a temperature of 25 ± 1 °C to a height of 30 cm. The disease model mice produced in Example 3-1 above were placed one by one in a water tank and tested for a total of 6 minutes. The first 2 minutes were not measured as adaptation time, and the immobility time of the experimental animals was measured for the next 4 minutes. Immobility means standing upright and floating motionless, making only the minimum movement to expose only the head above the water.

### 3-4. Tail suspension test (TST)

According to the method of Stern et al. (Stern L, Chermat R, Thierry B, Simon P (1985) The tail suspension test: A new method for screening antidepressants in mice, Psychopharmacology, Vol.85; pp.367-370), A fixation apparatus was attached to the end of the mouse's tail about 1 cm, and then it was hung 50 cm away from the ground in a container with a diameter of 35 cm and a height of 50 cm. The immobility time of the experimental animals was measured for a total of 6 minutes.

The ratio of each measurement time in the experiments 3-2 to 3-4 above is shown in Table 2 below. In addition, the concentration of corticosterone in blood, which induces anxiety, in each mouse was also measured and shown in Table 2 below.

**[Table 2]**

| Experimental group | EPM test (%, time spent in open arm) | FST test (%, immobility time) | TST test (%, immbolity time) | Corticosterone in blood (ng/mL) |
|---|---|---|---|---|
| Normal group | 11.2 | 21.5 | 26.6 | 14.5 |
| Group administered with E. coli | 7.5 | 38.3 | 49.2 | 286.0 |
| E. coli + heat-killed NK112 | 10.3 | 25.7 | 34.3 | 122.1 |
| E. coli + NK112 cell wall component | 10.1 | 25.3 | 32.2 | 210.0 |
| E. coli + NK112 cytoplasmic component | 9.3 | 27.7 | 37.3 | 241.0 |
| E. coli + Fluoxetine | 9.8 | 32.1 | 33.5 | 202.5 |

As shown in Table 2 above, it was confirmed that the time and frequency spent in open arms in the elevated plus maze test was increased and the amount of corticosterone in blood was significantly reduced the group administered with the *Lactobacillus paracasei* NK112 strain compared to the group administered with Escherichia coli alone, indicating that the *Lactobacillus paracasei* NK112 strain had anti-anxiety efficacy. In addition, it was confirmed that the immobility time in the forced swimming test and tail suspension test was significantly reduced in the group administered with the *Lactobacillus paracasei* NK112 strain, indicating that the *Lactobacillus paracasei* NK112 strain had anti-depression efficacy.

From the above results, it can be seen that the *Lactobacillus paracasei* NK112 strain has an excellent effect of alleviating depression and anxiety.

### [Example 4]

### Confirmation of cognitive function improvement efficacy of NK112 strain (Escherichia coli-induced model)

Using the following experimental method, it was confirmed that whether memory was improved after the disease model mouse produced in Example 3-1 above was treated with the *Lactobacillus paracasei* NK112 strain. As a positive control group, 5 mg/kg of donepezil (DNZ), which is used as a therapeutic agent for dementia, was used.

### 4-1. Y-maze task

In order to evaluate spatial cognitive ability, a Y-maze task was performed. In a Y-shaped maze that has a length of 40 cm, a width of 4 cm, and a height of 12 cm and is closed on all sides, three paths were designated as A, B, and C. Then, a mouse was placed in the center and observed for 8 minutes, and the total number of entries entered was recorded. If the mouse entered three different branches in turn, the alternation number was counted as 1. If the mouse did not enter consecutively, the alternation number was not counted. The alternation number is defined as entering all three in turn, and the calculation for evaluation of spatial cognitive function was performed using the following equation. Spontaneous alternation (%) = (total number of alternation / (total number of entries - 2)) X 100

### 4-2. Novel object recognition test (NORT)

The mouse was moved to the behavior observation room and acclimatized for 5 minutes. Thereafter, in a 3-minute exposure trial, two identical objects (1 set) were placed in a box 30 cm apart, and the time the mouse recognized the objects for 3 minutes was measured. In the exposure trial one day later, one object that had been revealed during exposure and one new object replaced were prepared in the same box, and the time the mouse recognized them was measured. The recognition index (%) is converted into the preference for new objects (time to recognize novel objects/total recognition time) X 100.

The ratio of each measured behavior and time in the experiments of 4-1 and 4-2 above is shown in Table 3 below. Furthermore, the expression level of brain-derived neurotrophic factor (BDNF) measured by the method of Example 2 is also shown..

**[Table 3]**

| Experimental group | Y-maze task (%) | NORT test (%) | Intensity of BDNF/β-actin |
|---|---|---|---|
| Normal group | 67.6 | 70.9 | 0.41 |
| Group administered with Escherichia coli | 53.4 | 54.2 | 0.25 |
| E. coli + NK112 | 63.1 | 64.3 | 0.38 |
| Positive control group (E. coli + Donepezil) | 63.2 | 62.2 | 0.35 |

As shown in Table 3 above, in the group administered with the *Lactobacillus paracasei* NK112 strain, the spontaneous alternation, the ratio of novel object recognition time, and the expression level of brain-derived neurotrophic factor were increased to levels equivalent to or higher than those of the group administered with donepezil.

From the above results, it can be seen that the *Lactobacillus paracasei* NK112 strain has an excellent cognitive function improvement efficacy.

### [Example 5]

### Confirmation of cognitive function improvement efficacy of NK112 strain (scopolamine model)

In order to confirm the cognitive function improvement efficacy of the *Lactobacillus paracasei* NK112, the heat-killed NK112 and cell wall components were orally administered at 10⁹ cfu/mouse each for 5 days, and then an experiment was performed. One hour before the behavioral experiment, the lactic acid bacteria samples were orally administered. Thirty minutes before the behavioral experiment, scopolamine, a parasympathetic inhibitor, was injected to induce cognitive function decline. As a positive control group, donepezil 2 mg/kg was orally administered.

Behavioral change evaluation was conducted using a Y-maze task and a passive avoidance test, and the Y-maze task was conducted in the same manner as in Example 4 above.

### Passive avoidance test (PAT)

In order to evaluate fear-related learning and memory, a passive avoidance test (PAT) was conducted in a passive avoidance box for two days. The passive avoidance box is divided into a bright room and a dark room, and an electric shock can be applied to the floor of the dark room. A light of about 50 W was turned on on one side of a compartment divided by a partition, and a mouse was placed there. The mouse looked around. When the partition door was opened 10 seconds later, the mouse entered the dark compartment. As soon as the mouse entered the dark compartment, the partition door was closed, and an electric shock was given by applying a 0.5 mA current through the stainless grid on the floor for 3 seconds. When the mouse was placed back in the bright room 24 hours after the electric shock, the latency time in the bright room was measured.

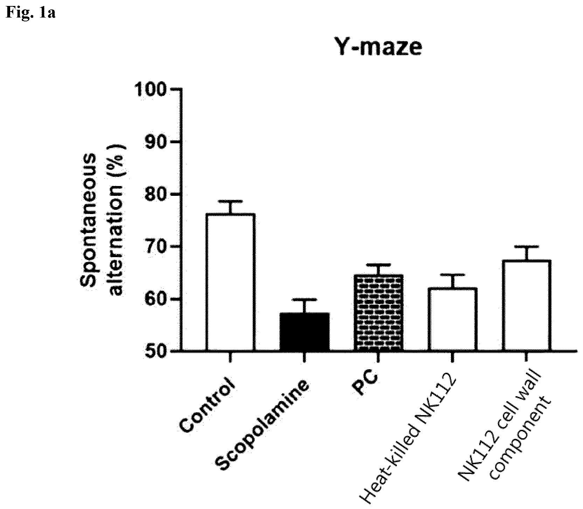

As shown in Figure 1a, as a result of the Y-maze task, the spontaneous alternation rate was 62.23% and 67.60%, respectively, in the group administered with heat-killed NK112 and the group administered with cell wall components, which were 8.45% and 17.81% higher than the group administered with scopolamine alone. The group administered with heat-killed NK112 and the group administered with cell wall components showed a similar or higher memory improvement effect compared to the group administered with donepezil, the positive control group (PC).

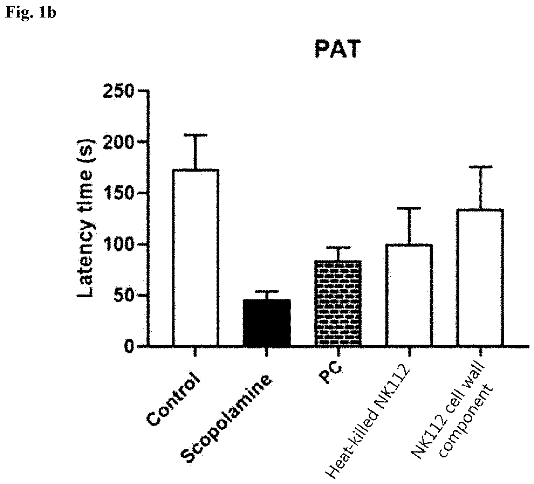

In addition, as shown in Figure 1b, as a result of the passive avoidance test, the latency time in a bright room due to fear memory was 101.22 seconds and 134.89 seconds, respectively, in the group administered with heat-killed NK112 and the group administered with cell wall components, which were 116.37 % and 188.35 % higher than the group administered with scopolamine alone, respectively. The values were 19.36% and 59.07% higher than the positive control group, respectively.

From this, it was confirmed that the *Lactobacillus paracasei* NK112 strain had an excellent improvement effect on memory decline.

### [Example 6]

### Confirmation of intestinal function improvement efficacy of NK112 strain

In order to evaluate the intestinal function improvement efficacy of *Lactobacillus paracasei* NK112, the disease model mouse produced in Example 3-1 above was anesthetized, and the intestinal length and inflammatory response indicators were measured.

### 6-1. Measurement of body weight and intestinal length

Changes in the mouse's body weight before and after the experiment were measured, the abdomen was dissected, the large intestine was separated, and the intestinal length was measured.

### 6-2. Measurement of myeloperoxidase (MPO) activity

200 µl of 10 mM potassium phosphate buffer (pH 7.0) containing 0.5% hexadecyl trimethyl ammonium bromide was added to 100 mg of the large intestine tissue and homogenized. The supernatant was obtained by centrifuging at 4 °C and 10,000 g for 10 minutes, which was used as a crude enzyme solution. 50 µl of the crude enzyme solution was added to 0.95 ml of a reaction solution containing 1.6 mM tetramethyl benzidine and 0.1 mM H₂O₂ and reacted at 37 °C, and the absorbance was measured over time at 650 nm. The activity of the myeloperoxidase was calculated as 1 unit for 1 µmol/ml of H₂O₂ produced as a reactant.

### 6-3. Measurement of p-p65, p65, and TNF-α

Inflammatory response indicators such as p-p65, p65, and TNF-α were measured. Specifically, the supernatant was obtained in the same manner as in Example 6-2, and 50 µg of the supernatant was taken and p-p65 and p65 were measured by immunoblotting. In addition, TNF-α was measured using an ELISA kit.

The results of measuring the improvement efficacy of *Lactobacillus paracasei* NK112 on E. coli-induced intestinal dysfunction are shown in Table 4 below.

**[Table 4]**

| Experimental group | Weight gain (g) | Intestinal length (cm) | MPO activity (µU/mg) | TNF-α (pg/mg) | Intensity of p-p65/p65 |
|---|---|---|---|---|---|
| Normal group | 0.65 | 6.30 | 0.54 | 36.00 | 0.21 |
| Group administered with Escherichia coli | -1.12 | 5.10 | 1.79 | 123.00 | 0.52 |
| E. coli+ heat-killed NK112 | 0.23 | 5.80 | 1.01 | 63.00 | 0.37 |
| E. coli + NK112 cell wall component | 0.22 | 5.80 | 1.00 | 61.00 | 0.37 |
| E. coli + NK112 cytoplasmic component | 0.22 | 5.70 | 1.10 | 78.00 | 0.43 |

As shown in Table 4, it was confirmed that when treated with *Lactobacillus paracasei* NK112, weight loss due to Escherichia coli administration was improved, intestinal length was restored, and levels of inflammatory indicators such as MPO activity, TNF-α, and p-p65/p65 were improved.

From this, it can be seen that the *Lactobacillus paracasei* NK112 strain has an excellent improvement effect on intestinal function.

### [Example 7]

### Preparation of the mixed composition comprising a Cuscuta australis R.Br. extract, a Cuscuta japonica Choisy extract, and a Lactobacillus paracasei NK112 strain

### 7-1. Preparation of single crude drug extract through ultrasonic extraction

In the following examples, the washed and dried *Cuscuta australis* R.Br. and *Cuscuta japonica* Choisy purchased from Jeongdo Herbal Medicine Co., Ltd., a Korean herbal medicine and food distributor, were used. Distilled water or 20% ethanol in an amount 10 times the amount of the raw material was added to 100 g of the raw material (whole crude drug) of the above-mentioned *Cuscuta australis* R.Br. and *Cuscuta japonica* Choisy, extracted at 25 °C for 1 hour, concentrated under reduced pressure at 50-65 °C, and then freeze-dried to obtain a total of 4 crude drug extract powders. The yields thereof are shown in Table 5 below.

**[Table 5]**

| | Solvent | Medicine | Yield (%) |
|---|---|---|---|
| Preparation Example 1-1 | distilled water | *Cuscuta australis* R.Br. | 5.0% |
| Preparation Example 1-2 | 20% ethanol | *Cuscuta australis* R.Br. | 4.5% |
| Preparation Example 1-3 | distilled water | *Cuscuta japonica* Choisy | 6.9% |
| Preparation Example 1-4 | 20% ethanol | *Cuscuta japonica* Choisy | 4.9% |

As can be seen in Table 5, it was confirmed that the yield of *Cuscuta australis* R.Br. and *Cuscutajaponica* Choisy was increased when distilled water was used compared to when 20% ethanol was used during ultrasonic extraction.

### 7-2. Preparation of single crude drug extract through reflux extraction

Distilled water or 20% ethanol in an amount 10 times the amount of the raw material was added to 100 g of the raw material (whole crude drug) of the *Cuscuta australis* R.Br. and *Cuscuta japonica* Choisy, reflux-extracted at 80-100 °C for 3 hours, concentrated under reduced pressure at 50-65 °C, and then freeze-dried to obtain a total of 4 crude drug extract powders. The yields thereof are shown in Table 6 below.

**[Table 6]**

| | Solvent | Medicine | Yield (%) |
|---|---|---|---|
| Preparation Example 2-1 | distilled water | *Cuscuta australis* R.Br. | 8.9% |
| Preparation Example 2-2 | 20% ethanol | *Cuscuta australis* R.Br. | 5.6% |
| Preparation Example 2-3 | distilled water | *Cuscuta japonica* Choisy | 6.6% |
| Preparation Example 2-4 | 20% ethanol | *Cuscuta japonica* Choisy | 5.2% |

As can be seen in Table 6, it was confirmed that the yield of *Cuscuta australis* R.Br. and *Cuscutajaponica* Choisy was increased when distilled water was used compared to when 20% ethanol was used during reflux extraction, and the yield of *Cuscuta australis* R.Br. and *Cuscuta japonica* Choisy was similar or higher when ultrasonic extraction in Example 7-1 was used compared to when reflux extraction was used.

### 7-3. Preparation of single crude drug extract according to grinding degree

Distilled water in an amount 10 times the amount of the raw material or powder was added to 100 g of the raw material (whole crude drug) and powder (powdered crude drug) of the *Cuscuta australis* R.Br. and *Cuscuta japonica* Choisy, reflux-extracted at 80-100 °C for 3 hours, concentrated under reduced pressure at 50-65 °C, and then freeze-dried to obtain a total of 4 crude drug extract powders. The yields thereof are shown in Table 7 below.

**[Table 7]**

| | Grinding or not | Medicine | Yield (%) | Remarks |
|---|---|---|---|---|
| Preparation Example 3-1 | whole crude drug | *Cuscuta australis* R.Br. | 8.9% | Same as in Preparation Example 2-1 |
| Preparation Example 3-2 | powdered crude drug | *Cuscuta australis* R.Br. | 16.4% | |
| Preparation Example 3-3 | whole crude drug | *Cuscuta japonica* Choisy | 6.6% | Same as in Preparation Example 2-3 |
| Preparation Example 3-4 | powdered crude drug | *Cuscuta japonica* Choisy | 16.9% | |

As can be seen in Table 7, it was confirmed that when reflux extraction was performed using distilled water, the yield was increased by about two times more when the powder of *Cuscuta australis* R.Br. or *Cuscuta japonica* Choisy was used than when the raw material of *Cuscuta australis* R.Br. or *Cuscuta japonica* Choisy was used.

### 7-4. Preparation of combined crude drug extract according to various solvent amounts

The powder (powdered crude drug) of *Cuscuta australis* R.Br. and *Cuscuta japonica* Choisy was mixed at a 3 : 1 weight ratio (w/w) to a total of 100 g, and then distilled water in an amount 10 times, 20 times 30 times, and 50 times the amount of the powder were added, reflux-extracted at 80-100 °C for 3 hours, concentrated under reduced pressure at 50-65 °C, and then freeze-dried to obtain a total of 4 crude drug extract powders. The yields thereof are shown in Table 8 below.

**[Table 8]**

| | Amount of solvent | Yield (%) |
|---|---|---|
| Preparation Example 4-1 | 10 times | 18.9% |
| Preparation Example 4-2 | 20 times | 27.4% |
| Preparation Example 4-3 | 30 times | 23.2% |
| Preparation Example 4-4 | 50 times | 18.4% |

As can be seen in Table 8, it was confirmed that when the powder of *Cuscuta australis* R.Br. and *Cuscuta japonica* Choisy was reflux-extracted using distilled water, the highest yield was obtained when extraction was performed by adding distilled water in an amount 20 to 30 times the amount of the powder.

### 7-5. NK112 strain and preparation of a mixed composition

The *Lactobacillus paracasei* NK112 strain was cultured in MRS medium, then centrifuged at 5,000 xg at 4 °C for 20 minutes, and then washed with physioligical saline. The heat-killed cells were prepared by heating twice for 30 minutes at 90 °C, and ultrasonic extraction was performed, and centrifugation was performed at 5,000 xg at 4 °C for 20 minutes, and the obtained precipitate was lyophilized to obtain the cell wall components (1 × 10⁹ CFU).

### [Example 8]

### Confirmation of nerve regeneration efficacy (ability to induce nerve growth factor (NGF) secretion) of the mixed composition

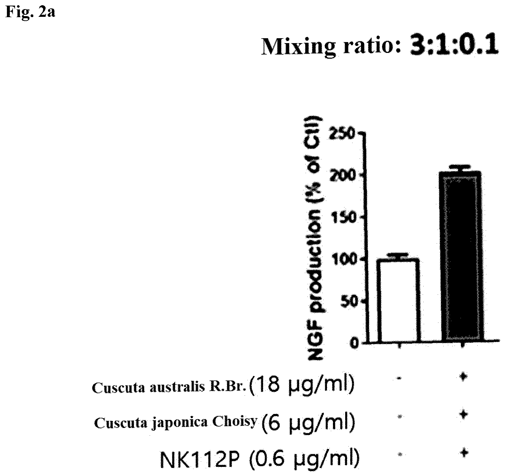

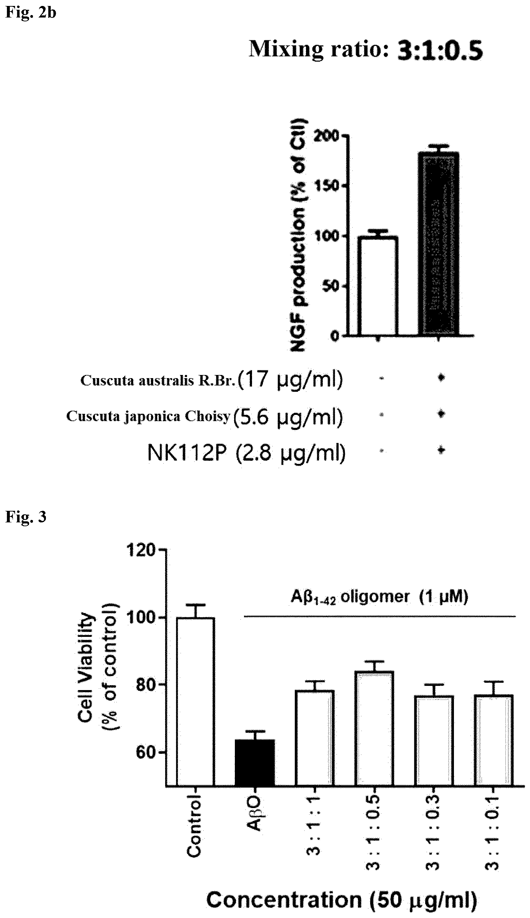

The N2a cells were dispensed in a 24 well plate at 1 × 10⁵ cells/well, and the cells were treated with a mixed composition of a *Cuscuta australis* R.Br. extract, a *Cuscuta japonica* Choisy extract, and NK112P at a ratio of 3 : 1 : 0.1 or 3 : 1 : 0.5 and cultured for 24 hours. Thereafter, the culture medium was collected, and the amount of nerve growth factor (NGF) secretion produced in the C6 glioma cell culture supernatant was quantified and measured using the Competitive Enzyme-Linked Immuno Assay (ELISA) kit (R&D systems, Minneapolis, MN, USA), an enzyme immunoassay. The amount of nerve growth factor (NGF) production (%) of the mixed composition was calculated compared to the amount of nerve growth factor production of the untreated control group (Ctl), and the results are shown in Figures 2a and 2b.

As shown in Figures 2a and 2b, the mixed composition of a *Cuscuta australis* R.Br. extract, a *Cuscuta japonica* Choisy extract, and NK112P at a ratio of 3 : 1 : 0.1 or 3 : 1 : 0.5 increased the amount of nerve growth factor production by 202.07% and 183.90% compared to the control group. Through this, it was found that the mixture comprising a *Cuscuta australis* R.Br. extract, a *Cuscuta japonica* Choisy extract, and a *Lactobacillus paracasei* NK112 strain has a nerve regeneration efficacy by inducing the secretion of nerve growth factors.

### [Example 9]

### Confirmation of anti-Alzheimer's efficacy (nerve cell protection efficacy) of the mixed composition

The HT22 cells were dispensed in a 96-well plate at 4 × 10⁴ cells/well, stabilized for 24 hours, then treated with a mixed composition of a *Cuscuta australis* R.Br. extract, a *Cuscuta japonica* Choisy extract, and NK112P at a ratio of 3 : 1 : 1, 3 : 1 : 0.5, 3 : 1 : 0.3 or 3 : 1 : 0.1 for 1 hour, then further treated with 3 µM of amyloid beta oligomer (AβO), a neurotoxin substance, and cultured for 23 hours. The medium was removed, and then the cells were treated with 1 mg/ml 3-(4,5-dimethylthiazol-2-yl)-2,5-diphenyltetrazolium bromide (MTT) solution for 4 hours, and the reduced formazan was dissolved in 100 µl of DMSO, and the cell viability was measured using a microspectrophotometer at a wavelength of 570 nm.

As shown in Figure 3, it was confirmed that the mixed composition of a *Cuscuta australis* R.Br. extract, a *Cuscuta japonica* Choisy extract, and NK112P exhibited excellent nerve cell protection effects compared to the control group at all mixing ratios. Specifically, in the case of the composition with at a ratio of 3 : 1 : 0.5, the cell viability was increased by 31.94 % compared to the control group. In the case of the composition with at a ratio of 3 : 1 : 1, the cell viability was increased by 20.96 % compared to the control group. In the case of the composition with at a ratio of 3 : 1 : 0.3, the cell viability was increased by 20.82 % compared to the control group. In the case of the composition with at a ratio of 3 : 1 : 0.1, the cell viability was increased by 20.96 % compared to the control group. Through this, it was found that the mixture comprising a *Cuscuta australis* R.Br. extract, a *Cuscuta japonica* Choisy extract, and a *Lactobacillus paracasei* NK112 strain protects nerve cells and exhibits excellent anti-Alzheimer's efficacy, and this efficacy is particularly excellent in a composition comprising a *Cuscuta australis* R.Br. extract, a *Cuscuta japonica* Choisy extract, and NK112 at a ratio of 3 : 1 : 0.5.

### [Example 10]

### Confirmation of cognitive function improvement efficacy of the mixed composition (scopolamine model)

In order to confirm the cognitive function improvement efficacy of the mixed composition of a *Cuscuta australis* R.Br. extract, a *Cuscuta japonica* Choisy extract, and NK112P at a ratio of 3 : 1 : 0.5, the composition was orally administered to mice for 5 days, and then a behavioral experiment was performed. One hour before the behavioral experiment, the composition was orally administered. Thirty minutes before the behavioral experiment, scopolamine was injected to induce cognitive function decline. As a positive control group, 2 mg/kg donepezil (DNZ) was orally administered.

Evaluation of behavioral change was conducted through a Y-maze task as in Example 4 above, a novel object recognition test, and a passive avoidance test as in Example 5 above.

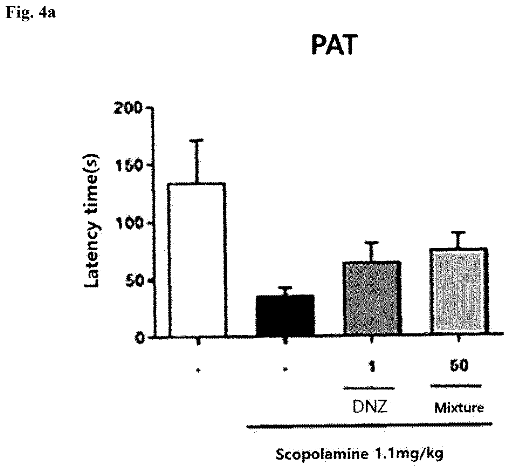

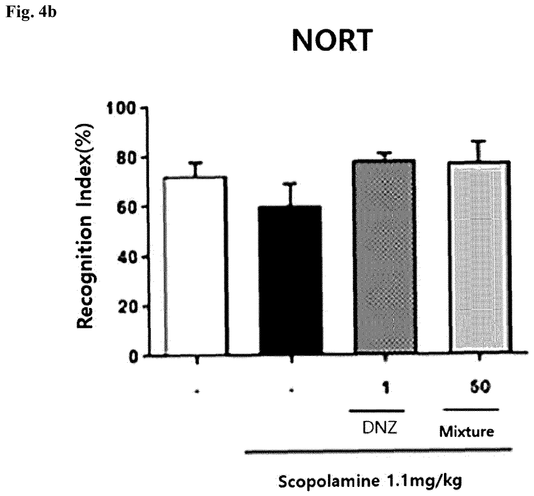

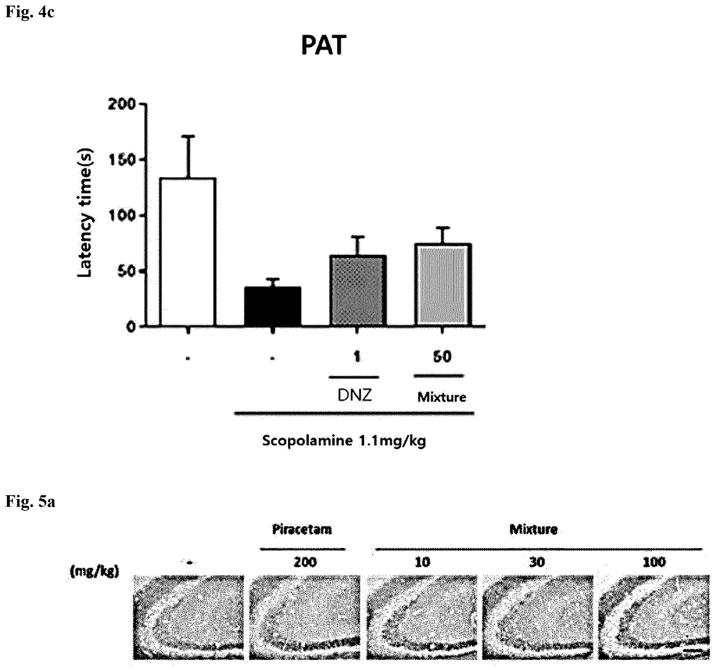

As shown in Figures 4a to 4c, as a result of measuring the spontaneous alternation in the Y-maze, the spontaneous alternation was 65.67 % in the group administered with the composition, which was 21.01 % higher than the group treated with scopolamine. As a result of measuring the recognition index in a novel object recognition test, the recognition index was 77.30 % in the group administered with the composition, which was 28.90 % higher than the group treated with scopolamine. As a result of measuring the latency time due to fear memory in a passive avoidance test, the latency time in the group administered with the composition was increased by 108.33 % compared to the group treated with scopolamine.

In addition, in all of the above experiments, the mixed composition of a *Cuscuta australis* R.Br. extract, a *Cuscuta japonica* Choisy extract, and NK112 exhibited equivalent or higher results compared to the group administered with donepezil, which was a positive control group. From this, it was confirmed that the mixed composition of a *Cuscuta australis* R.Br. extract, a *Cuscuta japonica* Choisy extract, and *Lactobacillus paracasei* NK112 had an excellent improvement effect on cognitive function decline.

### [Example 11]

### Confirmation of cognitive function improvement efficacy of the mixed composition (Escherichia coli-induced model)

In order to confirm the cognitive function improvement efficacy of the mixed composition of the *Cuscuta australis* R.Br. extract, a *Cuscuta japonica* Choisy extract, and NK112P at a ratio of 3 : 1 : 0.5, the mouse model of Example 3-1 above was subjected to a Y-maze task and a novel object recognition test in the same manner as in Example 4 above. In addition, the following Barnes maze task (BMT) was further performed.

### Barnes maze task (BMT)

The Barnes maze task is an experiment that measures learning ability and memory ability. The maze is a white disc with a diameter of 89 cm and a height of 140 cm, which has 20 avoidance holes of 5 cm in diameter. One avoidance hole has a black safety zone installed at the bottom so that it can be used as an avoidance place for experimental animals.

The disease model mouse produced in Example 3-1 above was placed in the center of the disk in the prepared experimental maze. Thereafter, a strong light was applied to measure the time it took for the mouse to avoid the light and find a dark safety zone, and this was repeated once a day for 4 days.

Additionally, the expression of brain-derived neurotrophic factor (BDNF) in the hippocampus and blood of mice was measured in the same manner as in Example 2 above, and the results are shown in Table 9 below.

**[Table 9]**

| Experimental group | Y-maze task (%) | NORT test (%) | BMT test (%, 4th day) | Intensity of BDNF/ β-actin |
|---|---|---|---|---|
| Normal group | 74.00 | 24.70 | 23.80 | 0.82 |
| Group administered with Escherichia coli | 51.20 | 9.00 | 54.60 | 0.32 |
| E. coli + composition (50 mg/kg) | 69.00 | 22.90 | 31.80 | 0.65 |
| E. coli + composition (10 mg/kg) | 63.60 | 20.60 | 37.20 | 0.46 |
| E. coli + composition (2 mg/kg) | 53.50 | 13.20 | 34.70 | 0.39 |

As shown in Table 9, it was confirmed that the group administered with the mixed composition of a *Cuscuta australis* R.Br. extract, a *Cuscuta japonica* Choisy extract, and NK112 showed improved results compared to the control group in the Y-maze task, novel object recognition test, and Barnes maze task, and the expression of brain-derived neurotrophic factor (BDNF), which decreases in aging and dementia, etc., was also increased in a dose-dependent manner. From this, it can be seen that the mixed composition of a *Cuscuta australis* R.Br. extract, a *Cuscuta japonica* Choisy extract, and *Lactobacillus paracasei* NK112 has excellent cognitive function improvement effects.

### [Example 12]

### Confirmation of synaptic strengthening efficacy of the mixed composition

The mixed composition of a *Cuscuta australis* R.Br. extract, a *Cuscuta japonica* Choisy extract, and NK112P at a ratio of 3 : 1 : 0.5 was orally administered to a mouse at 10, 30, or 100 mg/kg for 21 days, and the positive control group was intraperitoneally administered piracetam, an agent that improves symptoms of cognitive impairment, at 200 mg/kg. The mouse was perfused and fixed with paraformaldehyde, and the brain was extracted. The frozen-sectioning was performed, and then the hippocampus section was selected, washed three times with PBS, treated with hydrogen peroxide to remove endogenous peroxidase, and reacted with the primary antibody, mouse anti-synaptophysin antibody, overnight. Thereafter, it was reacted with the secondary antibody, biotinylated antimouse antibody, for 90 minutes, subjected to the ABC reaction (1 : 100 dilution) for 1 hour, and then color was developed using DAB solution (3,3-diaminobenzidine tetrahydrochloride) for 3 minutes. Washing was performed three times with PBS between each process. After the staining reaction, the tissue was placed on a gelatin-coated slide, dehydrated with 70-100% ethanol and xylene, and then covered with a cover slide for storage.

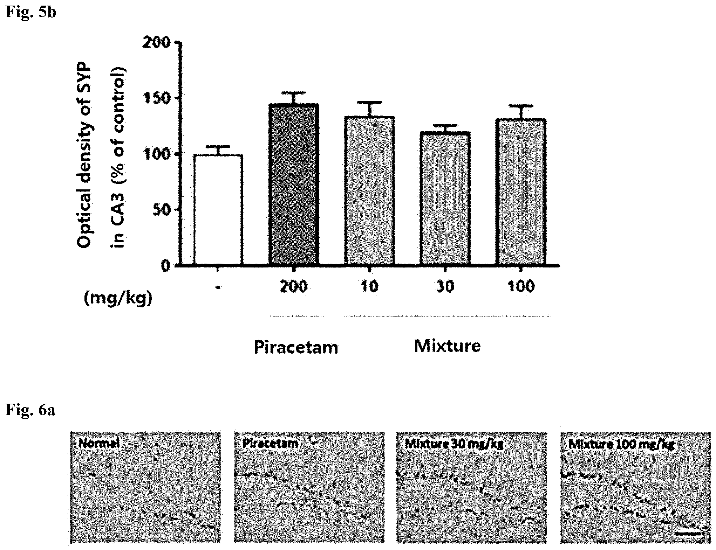

The stained brain tissue was observed in the Cornu Ammonis area of the hippocampus using an optical microscope (Olympus Microscope System BX51; Olympus, Tokyo, Japan). The photograph is shown in Figure 5a, and the amount of synaptic protein is shown in the graph of Figure 5b.

As shown in Figures 5a and 5b, the amount of synaptic protein in the group administered with the mixed composition of a *Cuscuta australis* R.Br. extract, a *Cuscuta japonica* Choisy extract, and NK112P at a ratio of 3 : 1 : 0.5 at a dose of 10, 30, and 100 mg/kg was increased by 134.6 %, 120.21 %, and 132.37 %, respectively, compared to that of normal mice. From this, it was confirmed that the mixed composition comprising a *Cuscuta australis* R.Br. extract, a *Cuscuta japonica* Choisy extract, and *Lactobacillus paracasei* NK112 was excellent in improving cognitive function and memory by promoting synapse formation.

### [Example 13]

### Confirmation of nerve regeneration efficacy (efficacy of increasing the number of neuroblast cells and increasing the length of neurites)

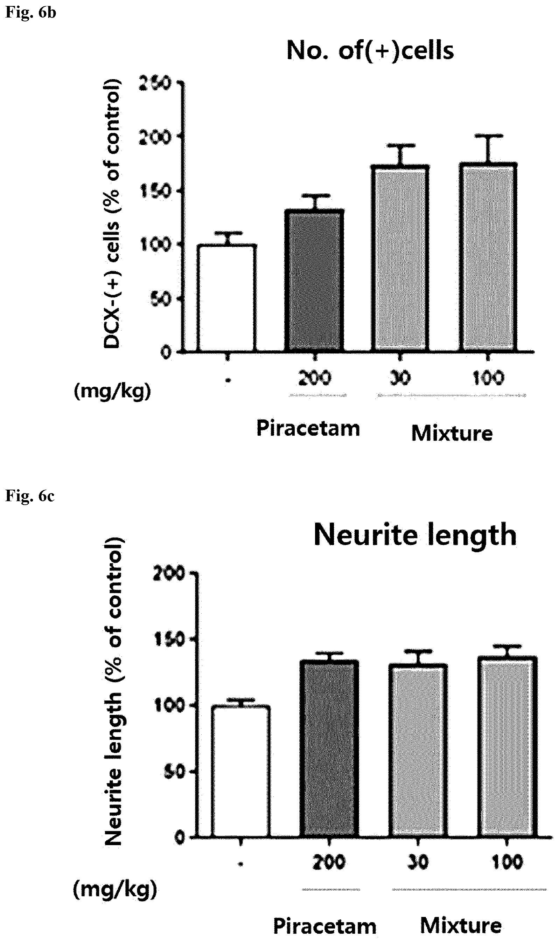

The experiment was performed as in Example 12 above, except that goat antidoublecortin (goat anti-DCX) antibody was used as the primary antibody and biotinylated anti-goat antibody was used as the secondary antibody. The stained brain tissue was observed as in Example 12, except that the effects on proliferation, neuronal differentiation, and neurite outgrowth of hippocampal dentate gyrus (DG) cells were observed. A photograph of doublecortin-positive cells observed at 200x magnification is shown in Figure 6a, and the number of neuroblast cells and the length of neurites are shown in Figures 6b and 6c.

As shown in Figure 6b, it was found that in the groups administered with the composition at 30 and 100 mg/kg, the number of neuroblasts was increased by 173.63% and 175.42%, respectively, compared to normal mice, and these values were 30.38% and 31.73% higher than the positive control group, the group administered with piracetam, respectively. In addition, as shown in Figure 6c, the neurite length of neuroblasts in the group administered with the composition was increased by 132.27% and 137.82%, respectively, which were similar levels to the positive control group.

From this, it can be seen that the mixed composition of a *Cuscuta australis* R.Br. extract, a *Cuscuta japonica* Choisy extract, and *Lactobacillus paracasei* NK112 has an excellent effect on improving cognitive function by promoting the proliferation of nerve cells.

### [Example 14]

### Confirmation of intestinal function improvement efficacy of the mixed composition

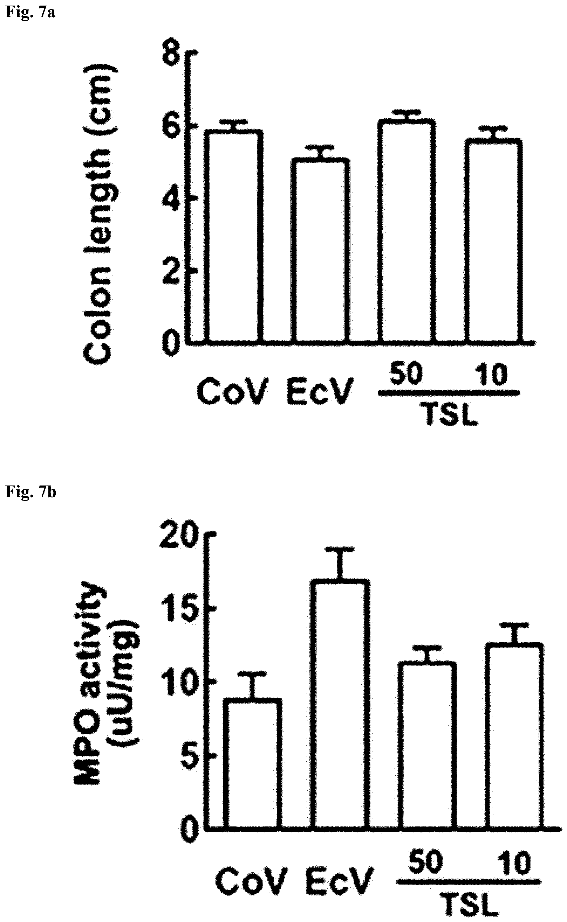

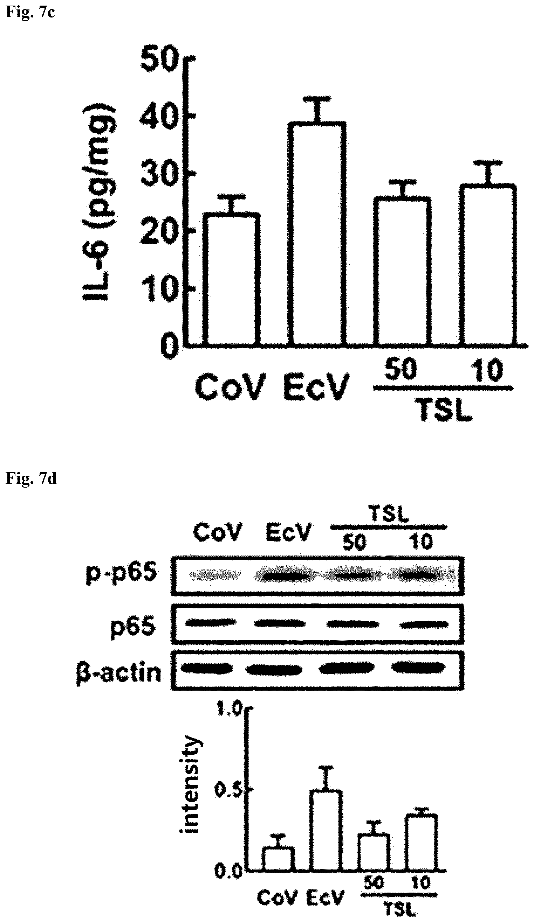

The mice that underwent behavioral evaluation in Example 11 above were anesthetized, and intestinal length and inflammatory response indicators were measured in the same manner as in Example 6 above, and the results are shown in Figures 7a to 7d.

As shown in Figures 7a to 7d, it was confirmed that in the group administered with Escherichia coli (EcV), intestinal length was reduced and the inflammatory index values such as MPO activity, TNF-α, IL-6, and p65 were worsened, but in the group administered with the mixed composition of a *Cuscuta australis* R.Br. extract, a *Cuscuta japonica* Choisy extract, and NK112P at a ratio of 3 : 1 : 0.5 at 10 or 50 mg/kg along with Escherichia coli (TSL), the intestinal length was restored to a level similar to that of the normal group (CoV), and the inflammatory index values were improved.

Therefore, it can be seen that the mixed composition of a *Cuscuta australis* R.Br. extract, a *Cuscuta japonica* Choisy extract, and *Lactobacillus paracasei* NK 112 has excellent intestinal function improvement effects.

## Claims

1. A *Lactobacillus paracasei* NK112 strain deposited under the accession number: KCCM12566P.

2. The *Lactobacillus paracasei* NK112 strain according to claim 1, wherein it has efficacy in preventing or treating cognitive dysfunction or intestinal dysfunction.

3. The *Lactobacillus paracasei* NK112 strain according to claim 1, wherein the cognitive dysfunction is at least one selected from the group consisting of Alzheimer's disease, schizophrenia, Parkinson's disease, Huntington's disease, Pick's disease, Creutzfeldt-Jakob disease, depression, anxiety, stroke, cerebral ischemia, encephalitis, amnesia, traumatic brain injury, Wernicke Korsakoff syndrome, epilepsy, seizure, hippocampal sclerosis, cerebral senescence, dementia, frontotemporal degeneration, normal pressure hydrocephalus, memory loss, and learning disability.

4. The *Lactobacillus paracasei* NK112 strain according to claim 1, wherein the intestinal dysfunction is at least one selected from the group consisting of inflammatory bowel disease, intestinal peristalsis disorder, intestinal obstruction, colon atony, irritable bowel syndrome, dyspepsia, abdominal distension, diarrhea, and constipation.

5. A pharmaceutical composition for prevention or treatment of cognitive dysfunction or intestinal dysfunction, comprising at least one selected from the group consisting of a *Lactobacillus paracasei* NK112 strain, a *Cuscuta australis* R.Br. extract, and a *Cuscuta japonica* Choisy extract.

6. The pharmaceutical composition for prevention or treatment of cognitive dysfunction or intestinal dysfunction according to claim 5, wherein the pharmaceutical composition comprises a *Lactobacillus paracasei* NK112 strain, a *Cuscuta australis* R.Br. extract, and a *Cuscuta japonica* Choisy extract.

7. The pharmaceutical composition for prevention or treatment of cognitive dysfunction or intestinal dysfunction according to claim 6, wherein the pharmaceutical composition comprises a *Cuscuta australis* R.Br. extract, a *Cuscuta japonica* Choisy extract, and a *Lactobacillus paracasei* NK112 strain at a weight ratio of 1 to 6 : 1 : 0.1 to 1.

8. The pharmaceutical composition for prevention or treatment of cognitive dysfunction or intestinal dysfunction according to claim 6, wherein the pharmaceutical composition comprises a *Cuscuta australis* R.Br. extract, a *Cuscuta japonica* Choisy extract, and a *Lactobacillus paracasei* NK112 strain at a weight ratio of 2 to 4 : 1 : 0.3 to 0.7.

9. The pharmaceutical composition for prevention or treatment of cognitive dysfunction or intestinal dysfunction according to claim 5, wherein the pharmaceutical composition promotes the expression of brain-derived neurotrophic factor (BDNF).

10. The pharmaceutical composition for prevention or treatment of cognitive dysfunction or intestinal dysfunction according to claim 5, wherein the pharmaceutical composition promotes the expression of nerve growth factor (NGF).

11. The pharmaceutical composition for prevention or treatment of cognitive dysfunction or intestinal dysfunction according to claim 5, wherein the cognitive dysfunction is at least one selected from the group consisting of Alzheimer's disease, schizophrenia, Parkinson's disease, Huntington's disease, Pick's disease, Creutzfeldt-Jakob disease, depression, anxiety, stroke, cerebral ischemia, encephalitis, amnesia, traumatic brain injury, Wernicke Korsakoff syndrome, epilepsy, seizure, hippocampal sclerosis, cerebral senescence, dementia, frontotemporal degeneration, normal pressure hydrocephalus, memory loss, and learning disability.

12. The pharmaceutical composition for prevention or treatment of cognitive dysfunction or intestinal dysfunction according to claim 5, wherein the intestinal dysfunction is at least one selected from the group consisting of inflammatory bowel disease, intestinal peristalsis disorder, intestinal obstruction, colon atony, irritable bowel syndrome, dyspepsia, abdominal distension, diarrhea, and constipation.

13. A food composition for prevention or alleviation of cognitive dysfunction or intestinal dysfunction, comprising at least one selected from the group consisting of a *Lactobacillus paracasei* NK112 strain, a *Cuscuta australis* R.Br. extract, and a *Cuscuta japonica* Choisy extract.

14. The food composition for prevention or alleviation of cognitive dysfunction or intestinal dysfunction according to claim 13, wherein the food composition comprises a *Lactobacillus paracasei* NK112 strain, a *Cuscuta australis* R.Br. extract, and a *Cuscuta japonica* Choisy extract.

15. An animal feed composition for prevention or alleviation of cognitive dysfunction or intestinal dysfunction, comprising at least one selected from the group consisting of a *Lactobacillus paracasei* NK112 strain, a *Cuscuta australis* R.Br. extract, and a *Cuscuta japonica* Choisy extract.

16. The animal feed composition for prevention or alleviation of cognitive dysfunction or intestinal dysfunction according to claim 15, wherein the animal feed composition comprises a *Lactobacillus paracasei* NK112 strain, a *Cuscuta australis* R.Br. extract, and a *Cuscutajaponica* Choisy extract.
